# EUROPEAN PATENT SPECIFICATION

(11) **EP 2 155 715 B1**
(45) Date of publication and mention of the grant of the patent: **13.03.2013**
(21) Application number: 08769462.6
(22) Date of filing: 13.05.2008
(51) Int. Cl.: C07D 401/06, C07D 409/06, A61K 31/4178, A61P 29/00

(54) **((PHENYL)IMIDAZOLYL)METHYLHETEROARYL COMPOUNDS**
((PHENYL)IMIDAZOLYL)METHYLHETEROARYL-VERBINDUNGEN
COMPOSÉS DE ((PHÉNYL)IMIDAZOLYL)MÉTHYLHÉTÉROARYLE

(30) Priority: 14.05.2007 US 917833 P
(43) Date of publication of application: 24.02.2010
(73) Proprietor: ALLERGAN, INC., Irvine, CA 92612 (US)
(72) Inventor: SINHA, Santosh, C., Ladera Ranch, CA 92694 (US); HEIDELBAUGH, Todd, M., Fountain Valley, CA 91708 (US); CHOW, Ken, Newport Coast, CA 92657 (US); BHAT, Smita, S., Irvine, CA 92614 (US); NGUYEN, Phong, X., Placentia, CA 92870 (US); GARST, Michael, E., Newport Beach, CA 92660 (US)
(74) Representative: HOFFMANN EITLE
(86) International application number: PCT/US2008/063489
(87) International publication number: WO 2008/141312

(56) References cited:
- WO-A-01/00586
- WO-A-97/35858
- BOYD RE ET AL: "alpha-2 adrenoceptor agonists as potential analgesic agents. 1. (Imidazolylmethyl)oxazoles and -thiazoles" JOURNAL OF MEDICINAL CHEMISTRY, vol. 42, 1999, pages 5064-5071, XP002492525
- LALCHANDANI SHILPA G ET AL: "Medetomidine analogs as selective agonists for the human .alpha.2-adrenoceptors" BIOCHEMICAL PHARMACOLOGY, PERGAMON, OXFORD, GB, vol. 67, no. 1, 1 January 2004 (2004-01-01), pages 87-96, XP002472947 ISSN: 0006-2952
- HONG S-S ET AL: "A STRUCTURE-ACTIVITY RELATIONSHIP STUDY OF BENZYLIC MODIFICATIONS OF 4-Ú1-(1-NAPHTHYL)ETHYL 3/4 -1H-IMIDAZOLES ON ALPHA1- AND ALPHA2-ADRENERGIC RECEPTORS" JOURNAL OF MEDICINAL CHEMISTRY, US AMERICAN CHEMICAL SOCIETY. WASHINGTON, vol. 37, no. 15, 22 July 1994 (1994-07-22), pages 2328-2333, XP002022576 ISSN: 0022-2623
- YOSHIYA AMEMIYA ET AL: "Synthesis and alpha-adrenergic activities of 2- and 4-substituted imidazoline and imidazole analogues" JOURNAL OF MEDICINAL CHEMISTRY, US AMERICAN CHEMICAL SOCIETY. WASHINGTON, vol. 35, no. 4, 1 January 1992 (1992-01-01), pages 750-755, XP002151512 ISSN: 0022-2623
- ZHANG X ET AL: "Medetomidine Analogs as alpha 2-Adrenergic Ligands. 2. Design, Synthesis and Biological Activity of Conformationally Restricted Naphthalene Derivatives of Medetomidine" JOURNAL OF MEDICINAL CHEMISTRY, US AMERICAN CHEMICAL SOCIETY. WASHINGTON, vol. 39, 1 January 1996 (1996-01-01), pages 3001-3013, XP002217487 ISSN: 0022-2623

## Description

### Cross Reference

This application claims the benefit of U.S. Application serial number 60/917,833, filed May 14, 2007.

**Background**

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into α₁, α₂, β₁, and β₂ subtypes. Functional differences between α₁ and α₂ receptors have been recognized, and compounds which exhibit selective binding between these two subtypes have been developed. Thus, in published international patent application WO 92/0073. the selective ability of the R(+) enantiomer of terazosin to selectively bind to adrenergic receptors of the α₁ subtype was reported. The α₁/α₂ selectivity of this compound was disclosed as being significant because agonist stimulation of the α₂ receptors was said to inhibit secretion of epinephrine and norepinephrine, while antagonism of the α₂ receptor was said to increase secretion of these hormones. Thus, the use of non-selective alpha-adrenergic blockers, such as phenoxybenzamine and phentolamine, was said to be limited by their α₂ adrenergic receptor mediated induction of increased plasma catecholamine concentration and the attendant physiological sequelae (increased heart rate and smooth muscle contraction). For a further general background on the α-adrenergic receptors, the reader's attention is directed to Robert R. Ruffolo, Jr., α-Adrenoreceptors: Molecular Biology, Biochemistry and Pharmacology, (Progress in Basic and Clinical Pharmacology series, Karger, 1991), wherein the basis of α₁/α₂ subclassification, the molecular biology, signal transduction, agonist structure-activity relationships, receptor functions, and therapeutic applications for compounds exhibiting α-adrenergic receptor affinity is explored.

The cloning, sequencing and expression of alpha receptor subtypes from animal tissues has led to the subclassification of the α₁ adrenoreceptors into α_{1A}, α_{1B}, and α_{1D}. Similarly, the α₁ adrenoreceptors have also been classified α_{1A}, α_{2B}, and α_{2C} receptors. Each α₂ receptor subtype appears to exhibit its own pharmacological and tissue specificities. Compounds having a degree of specificity for one or more of these subtypes may be more specific therapeutic agents for a given indication than an α₂ receptor pan-agonist (such as the drug clonidine) or a pan-antagonist.

Among other indications, such as the treatment of glaucoma, hypertension, sexual dysfunction, and depression, certain compounds having alpha₂ adrenergic receptor agonist activity are known analgesics. However, many compounds having such activity do not provide the activity and specificity desirable when treating disorders modulated by alpha₂ adrenoreceptors. For example, many compounds found to be effective agents in the treatment of pain are frequently found to have undesirable side effects, such as causing hypotension and sedation at systemically effective doses. There is a need for new drugs that provide relief from pain without causing these undesirable side effects. Additionally, there is a need for agents which display activity against pain, particularly chronic pain, such as chronic neuropathic and visceral pain.

**Description of the Invention**

**Disclosed herein is a compound of the formula** 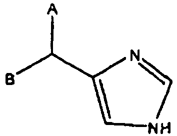 wherein
A is pyridyl, thienyl, furyl, pyrrolyl, pyrrolidinyl, imidazolyl, oxazolyl, pyrazolyl, pyrimidinyl, or pyrazinyl having 0, 1, 2, or 3 substituents;
B is phenyl having 0, 1, 2, or 3 substituents; and
each substituent is independently alkyl having from 1 to 8 carbon atoms, -F, -Cl, -Br, -CH₂OH, aminomethyl, -CH₂CN, -CF₃, or acyl having from 1 to 8 carbons.

Unless otherwise indicated, reference to a compound should be construed broadly to include pharmaceutically acceptable salts, tautomers, alternate solid forms, and non-covalent complexes of a chemical entity of the depicted structure or chemical name.

A pharmaceutically acceptable salt is any salt that retains the activity of the parent compound and does not additional unacceptable deleterious or untoward effects on the subject to which it is administered and in the context in which it is administered compared to the parent compound. A pharmaceutically acceptable salt also refers to any salt which may form in vivo as a result of administration of an acid, another salt, or a prodrug which is converted into an acid or salt.

Pharmaceutically acceptable salts of acidic functional groups may be derived from organic or inorganic bases. The salt may comprise a mono or polyvalent ion. Of particular interest are the inorganic ions, lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Hydrochloric acid or some other pharmaceutically acceptable acid may form a salt with a compound that includes a basic group, such as an amine or a pyridine ring.

Tautomers are isomers that are in rapid equilibrium with one another. They often, but do not necessarily, include a transfer of a proton, hydrogen atom, or hydride ion. For example, the structures herein are intended to include, but are not limited to, the tautomeric forms shown below.

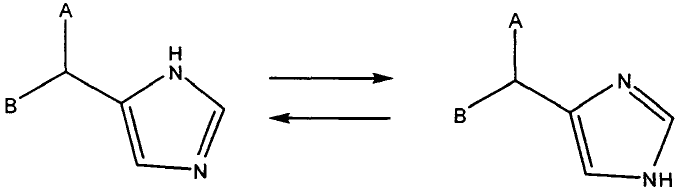

Unless stereochemistry is explicitly depicted, a structure is intended to include every possible stereoisomer, both pure or in any possible mixture.

Alternate solid forms are different solid forms than ones that may result from practicing the procedures described herein. For example, alternate solid forms may be polymorphs, different kinds of amorphous solid forms, glasses, and the like.

Non-covalent complexes are complexes that may form between the compound and one or more additional chemical species that do not involve a covalent bonding interaction between the compound and the additional chemical species. They may or may not have a specific ratio between the compound and the additional chemical species. Examples might include solvates, hydrates, charge transfer complexes, and the like.

A is pyridyl, thienyl, furyl, pyrrolyl, pyrrolidinyl, imidazolyl, oxazolyl, pyrazolyl, pyrimidinyl, or pyrazinyl. A can be unsubstituted, or substituted with up to 3 substituents.

B is phenyl which is unsubstituted or has up to 3 substituents.

The substituents of A and B are subject to the same constraints. However, the substituents are independent, meaning that A and B may have the same or different substituents; the substituents on A may be the same or different from one another; and the substituents on B may be the same or different from one another.

Examples of substituents include:

a. alkyl, meaning hydrocarbyl having no double or triple bonds, including, but not limited to:
   - linear alkyl, e.g. methyl, ethyl, n-propyl, n-butyl, n-pentyl, n-hexyl, etc.,
   - branched alkyl, e.g. iso-propyl, t-butyl and other branched butyl isomers, branched pentyl isomers, etc.,
   - cycloalkyl, e.g. cyclopropyl, cyclobutyl, cyclopentyl, cyclohexyl, etc.,
   - combinations of linear, branched, and/or cycloalkyl;

-CH₂-CN;

hydroxymethyl;

aminomethyl (-CH₂-amine);

acyl (i.e. 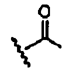 hydrocarbyl), and in particular, acetyl, propionyl, and benzoyl substituents are contemplated; and

-CF₃.

Alternatively, a substituent may be -F, -Cl, or -Br,

In particular, alkyl having from 1 to 8 carbon atoms is contemplated as a substituent.

Alternatively, alkyl having from 1 to 4 carbon atoms is contemplated;

Substituents must be sufficiently stable to be stored in a bottle at room temperature under a normal atmosphere for at least 12 hours, or stable enough to be useful for any purpose disclosed herein.

In one embodiment, A is pyridinyl, meaning that compounds of structures such as those shown below are contemplated.

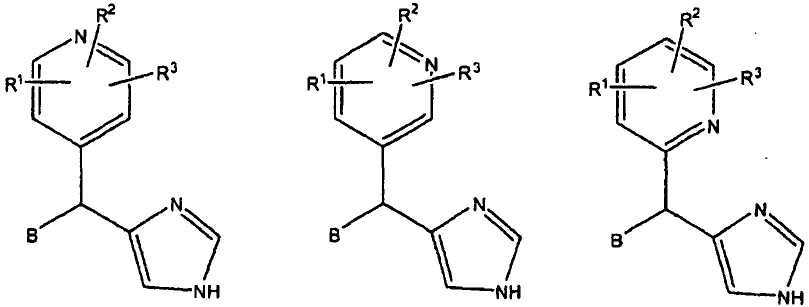

In these structures, R¹, R², and R³ are substituents as defined herein.

In another embodiment, A is thienyl, meaning that compounds of structures such as those shown below are contemplated.

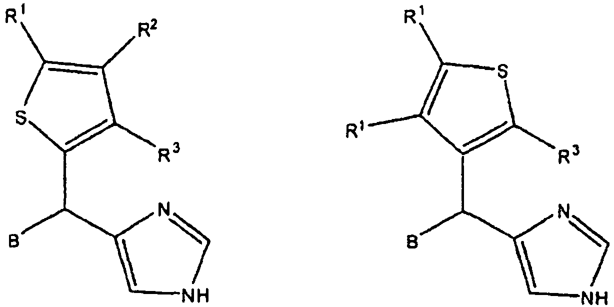

In these structures, R¹, R², and R³ are substituents as defined herein.

In another embodiment, A is furyl, meaning that compounds of structures such as those shown below are contemplated.

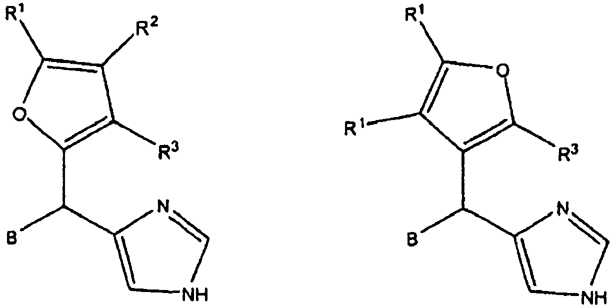

In these structures, R¹,R², and R³ are hydrogen or substituents wherein each substituent is independently alkyl having from 1 to 8 carbon atoms,-F, -Cl, -Br, -CH₂OH, aminomethyl, -CH₂CN, -CF₃, or acyl having from 1 to 8 carbons.

In another embodiment, A is unsubstituted or has an isopropyl substituent.

In another embodiment, each substituent of B is -F, -Cl, -CH₃, or -CF₃.

A is pyridyl, thienyl, furyl, pyrrolyl, pyrrolidinyl, imidazolyl, oxazolyl, pyrazolyl, pyrimidinyl, or pyrazinyl having 0, 1, 2, or 3 substituents.

In another embodiment, A is pyridinyl,

In another embodiment, A is thienyl.

Another embodiment is a compound disclosed herein for use in the treatment of pain in a mammal in need thereof.

Another embodiment is use of a compound disclosed herein in the manufacture of a medicament for the treatment of pain.

Another embodiment is a pharmaceutically acceptable composition comprising disclosed herein.

Another embodiment is a kit comprising a package containing the composition disclosed herein and a label indicating the use of the composition for the treatment of pain.

Some hypothetical examples of useful compounds are depicted below.

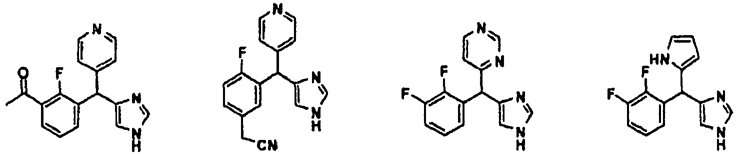

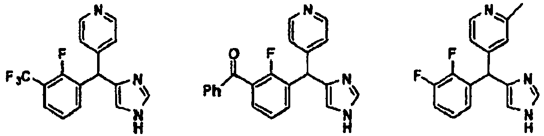

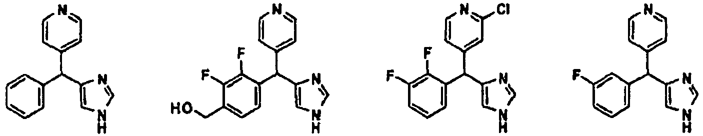

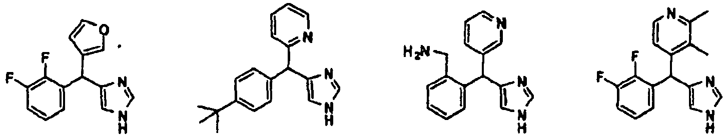

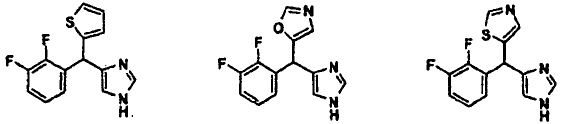

The compounds disclosed herein are useful for treating neurological conditions of conditions and diseases which are responsive to treatment by alpha₂ adrenergic agonists. Such conditions and diseases include, but are not limited to, pain including chronic pain (which may be, without limitation visceral, inflammatory, referred or neuropathic in origin) neuropathic pain, corneal pain, glaucoma, reducing elevated intraocular pressure, ischemic neuropathies and other neurodegenerative diseases, diarrhea, and nasal congestion. Chronic pain may arise as a result of, or be attendant to, conditions including without limitation: arthritis, (including rheumatoid arthritis), spondylitis, gouty arthritis, osteoarthritis, juvenile arthritis, and autoimmune diseases including without limitation, lupus erythematosus. Visceral pain may include, without limitation, pain caused by cancer or attendant to the treatment of cancer as, for example, by chemotherapy or radiation therapy. In addition, the compounds disclosed herein are useful for treating muscle spasticity including hyperactive micturition, diuresis, withdrawal syndromes, neurodegenerative diseases including optic neuropathy, spinal ischemia and stroke, memory and cognition deficits, attention deficit disorder, psychoses including manic disorders, anxiety, depression, hypertension, congestive heart failure, cardiac ischemia and nasal congestion, chronic gastrointestinal inflammations, Crohn's disease, gastritis, irritable bowel syndrome (IBS), functional dyspepsia and ulcerative colitis. The activity of the alpha_{2B/2C} specific or selective compounds disclosed herein is highly advantageous because the administration of these compounds to mammals does not result in sedation or in significant cardivascular effects (such as changes in blood pressure or heart rate).

It is known that chronic pain (such as pain from cancer, arthritis, and many neuropathic injuries) and acute pain (such as that pain produced by an immediate mechanical stimulus, such as tissue section, pinch, prick, or crush) are distinct neurological phenomena mediated to a large degree either by different nerve fibers and neuroreceptors or by a rearrangement or alteration of the function of these nerves upon chronic stimulation. Sensation of acute pain is transmitted quite quickly, primarily by afferent nerve fibers termed C fibers, which normally have a high threshold for mechanical, thermal, and chemical stimulation. While the mechanisms of chronic pain are not completely understood, acute tissue injury can give rise within minutes or hours after the initial stimulation to secondary symptoms, including a regional reduction in the magnitude of the stimulus necessary to elicit a pain response. This phenomenon, which typically occurs in a region emanating from (but larger than) the site of the original stimulus, is termed hyperalgesia. The secondary response can give rise to profoundly enhanced sensitivity to mechanical or thermal stimulus.

The A afferent fibers (Aβ and A fibers) can be stimulated at a lower threshold than C fibers, and appear to be involved in the sensation of chronic pain. For example, under normal conditions, low threshold stimulation of these fibers (such as a light brush or tickling) is not painful. However, under certain conditions such as those following nerve injury or in the herpes virus-mediated condition known as shingles the application of even such a light touch or the brush of clothing can be very painful. This condition is termed allodynia and appears to be mediated at least in part by Aβ afferent nerves. C fibers may also be involved in the sensation of chronic pain, but if so it appears clear that persistent firing of the neurons over time brings about some sort of change which now results in the sensation of chronic pain.

By "acute pain" is meant immediate, usually high threshold, pain brought about by injury such as a cut, crush, burn, or by chemical stimulation such as that experienced upon exposure to capsaicin, the active ingredient in chili peppers.

By "chronic pain" is meant pain other than acute pain, such as, without limitation, neuropathic pain. visceral pain (including that brought about by Crohn's disease and irritable bowel syndrome (IBS)), and referred pain.

For the purposes of this disclosure, "treat," "treating," or "treatment" refer to the use of a compound, composition, therapeutically active agent, or drug in the diagnosis, cure, mitigation. treatment, prevention of disease or other undesirable condition.

These compounds may be formulated into solid, liquid, or other types of dosage forms using methods known in the art. Both formulation of dosage forms and determination of a therapeutically effective dose can be readily made by a person of ordinary skill using routine methods.

**GENERAL METHODS FOR OBTAINING THE COMPOUNDS**

**Reaction Schemes A** and **B** illustrate general methods for obtaining the biaryl imidazole.

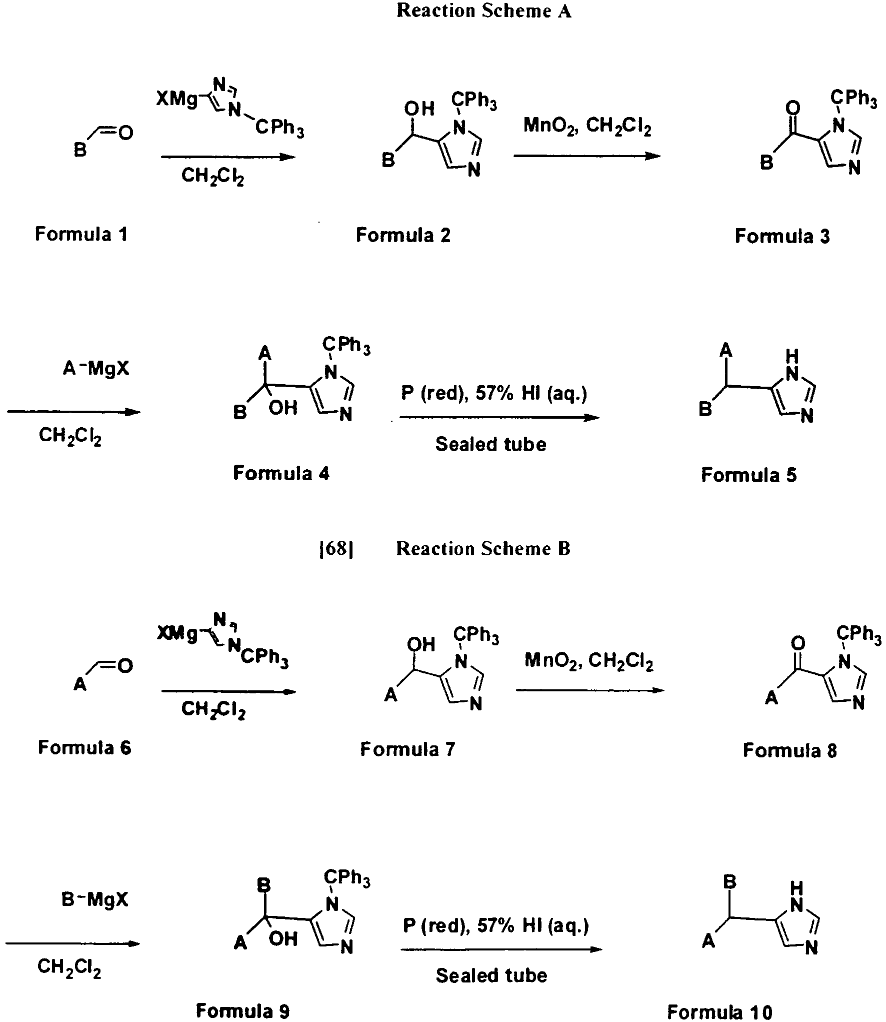

**SPECIFIC EMBODIMENTS, EXPERIMENTAL**

**Example A**

**Method A: Procedure for the preparation of 2-((3-chloro-2-fluorophenyl)(1H-imidazol-5-yl)methyl)pyridine (658)**

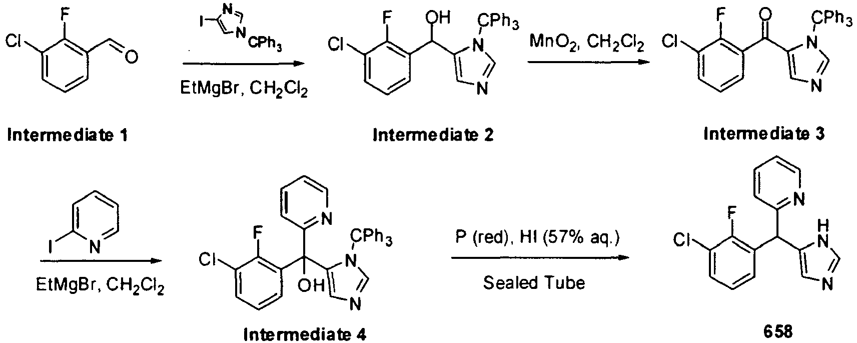

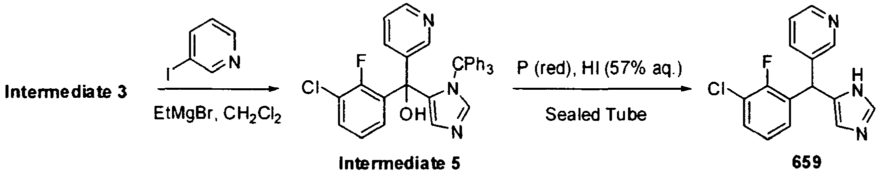

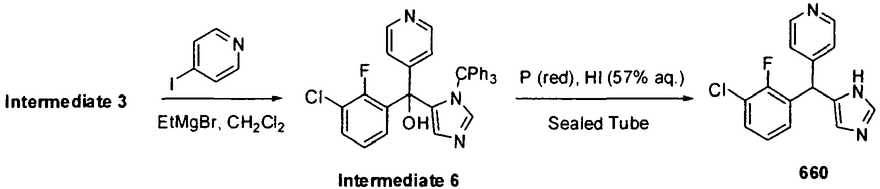

A solution of 4-iodo-1-tritylimidazole (commercially available, 5.1 g, 11.8 mmol) in dichloromethane (40 mL) at -10°C was treated with ethyl magnesium bromide (3.9 mL, 11.8 mmol, 3M in ether) and allowed to react for 45 m. A solution of 3-chloro-2-fluoro-benzaldehyde, **(intermediate 1)** (1.5 g, 9.4 mmol) in dichloromethane was added via syringe at -10°C and stirred for 16 hours at room temperature. The mixture was quenched with water (50 mL) and a saturated solution of ammonium chloride (50 mL). The residue was isolated in a typical aqueous workup and purified by MPLC with 3 to 5 % MeOH:CH₂Cl₂ to give (3-chloro-2-fluorophenyl)(1-trityl-1H-imidazol-4-yl) methanol, (**Intermediate 2**) as a solid, (3.5 g 78.7%).

A mixture of (3-chloro-2-fluorophenyl)(1-trityl-1H-imidazol-4-yl) methanol, (**Intermediate 2**) (3.5 g, 7.4 mmol) in CH₂Cl₂ (60 mL) was treated with manganese(IV) oxide, activated (commercially available from Aldrich): MnO₂ (3.9 g, 44.8 mmol) at room temperature. The mixture was heated to 60 °C for 2 hours. The mixture was then cooled to room temperature and filtered through celite and the solvent was removed under vacuum. The residue was purified by MPLC with 3 to 5 % MeOH :CH₂Cl₂ to give (3-chloro-2-fluorophenyl)(1-trityl-1H-imidazol-5-yl)methanone, (**Intermediate 3**) (3.3 g, 97%).

A solution of 2-iodo-pyridine (commercially available) (415 mg, 2.02 mmol) in dichloromethane (25 mL) at room temperature was treated with ethyl magnesium bromide (0.67 mL, 2.02 mmol, 3M in ether) and allowed to react for 45 minutes. A solution of (3-chloro-2-fluorophenyl) (1-trityl-1H-imidazol-5-yl) methanone, (**Intermediate 3**) (630 mg, 1.35 mmol) in dichloromethane was added via syringe at room temperature and the reaction mixture was then stirred at room temperature for 16 hours. The mixture was quenched with water (50 mL) and a sat. solution of ammonium chloride (20 mL). The residue was isolated in a typical aqueous to give (3-chloro-2-fluorophenyl)(pyridin-2-yl)(1-trityl-1H-imidazol-5-yl)methanol, (**Intermediate 4**) (726 mg, crude).

A mixture of (3-chloro-2-fluorophenyl)(pyridin-2-yl)(1-trityl-1H-imidazol-5-yl)methanol, (**Intermediate 4**) (726g, 1.33 mmol) in 57% aqueous HI (10 mL) and iPrOH (2 mL) was added red phosphorus (412 mg, 13.3 mmol) in a resealable tube was heated at 160°C for 16 h. The mixture was then cooled to room temperature and poured into ice-water, which was then basified with NaOH and diluted with CHCl₃. The residue was isolated in a typical aqueous workup using CHCl₃ and purified by MPLC with 5 to 15 % MeOH:CH₂Cl₂ to give (3-chloro-2-fluorophenyl)(pyridin-2-yl)(1-trityl-1H-imidazol-5-yl)methanol, (**658**) as a solid, 236 mg (61% in two steps). ¹HNMR (CD₃OD, 300MHz) δ 8.47 (d, *J =* 4.4 Hz, 1H), 7.80 (dt, *J* = 2.1, 7.8 Hz, = I H), 7.76 (s, 1H), 7.39-7.23 (m, 3H), 7.12-7.00 (m, 2H), 6.60 (s, 1H), 5.86 (s, 1H).

Following a procedure similar to that for **658** afforded **659**, 3-((3-chloro-2-fluorophenyl)(1H-imidazol-5-yl)methyl)pyridine (162 mg, 44% yield in two steps) from (**Intermediate 3**) as a white solid. ¹HNMR (CD₃OD, 300MHz) δ 8.42 (d, *J* = 5.1 Hz, 1H), 8.38 (d, *J* = 2.1 Hz, 1H), 7.70 (s, 1H), 7.67-7.64 (m, 1H), 7.41-7.37 (m, 2H), 7.15-7.05 (m, 2H), 6.66 (s, 1H), 5.80 (s, I H).

Following a procedure similar to that for **658** afforded **660**, 4-((3-chloro-2-fluorophenyl)(1H-imidazo)-5-yl)methyl)pyridine (208 mg, 57.5% yield in two steps) from (**Intermediate 3**) as a white solid. ¹HNMR (CD₃OD, 300MHz) δ 8.47-8.45 (m, 2H), 7.70 (s, 1H), 7.42 (td, *J*= 1.8 Hz, 7.5 Hz, 1H), 7.37-7.24 (m, 2H), 7.15-7.03 (m, 2H), 6.69 (s, 1H), 5.78 (s, 1H).

**Example B**

**Method B: Procedure for the preparation of 3-((2,3-dimethylphenyl)(1H-imidazol-5-yl)methyl)pyridine (AGN213890)**

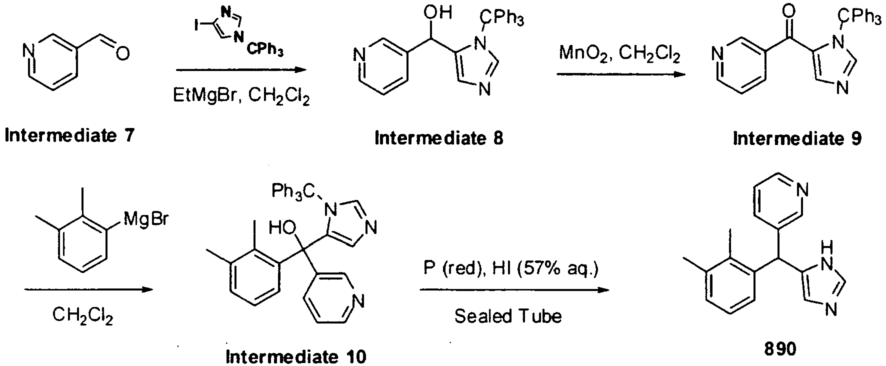

A mixture of 4-iodo-1-tritylimidazole (commercially available) (10.1 g, 23.3 mmol) in dichloromethane (100 mL) at -10 °C was treated with ethyl magnesium bromide (7.76 mL, 23.3 mmol, 3M in ether) and allowed to react for 45 minutes. A solution of 3-picolinaldehyde, (**intermediate 7**) (2 g, 18.69 mmol) in dichloromethane was added via syringe at -10 °C and stirred for 16 hours at room temperature. The mixture was quenched with water (50 mL) and a sat. solution of ammonium chloride (50 mL). The residue was isolated in a typical aqueous workup gave pyridin-3-yl(1-trityl-1H-imidazol-5-yl)methanol, (**intermediate 8**) as a solid, 6.48 g (82%).

A solution of pyridin-3-yl(1-trityl-1H-imidazol-5-yl)methanol, (**intermediate 8**) (6.48 g, 15.56 mmol) in CH₂Cl₂ (100 mL) was treated with manganese(IV) oxide, activated (commercially available from Aldrich) MnO₂ (8.1 g, 93.36 mmol) at room temperature. The mixture was heated to 60 °C for 2 hours. The mixture was cooled to room temperature and filtered through Celite and the solvent was removed under vacuum. The residue was purified by chromatography on silica gel with 5 % MeOH: CH₂Cl₂ to give pyridin-3-yl(1-trityl-1H-imidazol-5-yl)methanone. (**intermediate 9**) (6.1 g, 94.4%)

To a solution pyridin-3-yl(1-trityl-1H-imidazol-5-yl)methanone, (**intermediate 9**) (500 mg, 1.20 mmol) in dichloromethane (20 mL) at room temperature was treated with 2,3-dimethyl phenyl magnesium bromide (commercially available) (3.6 mL, 1.8 mmol, 0.5M in THF) and stirred at room temperature for 16 hours. The mixture was quenched with water (50 mL) and a sat. solution of ammonium chloride (50 mL). The residue was isolated in a typical aqueous workup to get (2,3-dimethylphenyl)(pyridin-3-yl)(1-trityl-1H-imidazol-5-yl)methanol, (**intermediate 10**) as a solid, 535 mg (crude).

A mixture of (3-chloro-2-fluorophenyl)(pyridin-3-yl)(1-trityl-1H-imidazol-5-yl)methanol, (**intermediate 10**) (535 mg, 1.02 mmol) in 57% aqueous HI (10 mL) was added red phosphorus (318 mg, 10.2 mmol) in a resealable tube was heated at 160 °C for 16 hours. The mixture was then cooled to room temperature and poured into ice-water, which was then basified with NaOH and diluted with CHCl₃. The residue was isolated in a typical aqueous workup and purified by chromatography on silica gel with 3 to 5 % NH₃-MeOH:CH₂Cl₂ to give (3-chloro-2-fluorophenyl)(pyridin-3-yl)(1-trityl-1H-imidazol-5-yl)methanol, (**890**) as a solid, (51 mg). ¹HNMR (CD₃OD, 300MHz): δ 8.39-8.3 (m, 1H), 8.29 (d, *J* = 2.4 Hz, 1H), 7.67 (s, 1H), 7.56-7.52 (m, 1H), 7.37-7.33 (m, 1H), 7.07-6.97 (m, 2H), 6.68 (d, *J*= 7.5 Hz, 1H), 6.40 (s, 1H), 5.76 (s, 1H), 2.27 (s, 3H), 2.16 (s, 3H).

The following compounds have been synthesized by one of the methods described above:

**2-((2,3-dimethylphenyl)(1H-imidazol-4-yl)methyl)pyridine,301:**

**(Method: B)**

¹H NMR (300 MHz, CD₃OD): δ 8.47 (d, *J* = 4.2 Hz, 1H), 7.89 (s, 1H), 7.83-7.72 (m, 1H), 7.31-7.27 (m, 1H), 7.11-6.96 (m, 3H), 6.66 (d, *J* = 7.5 Hz, I H), 6.45 (s, 1H), 5.85 (s, 1H), 2.28 (s, 3H), 2.16 (s, 3H).

**4-((2,3-dimethylphenyl)(1H-imidazol-5-yl)methyl)pyridine, 300:**

**(Method: B)**

**¹H NMR** (300 MHz, CD₃OD): δ 8.42-8.40 (m, 2H), 7.67 (s, 1H), 7.17-7.15 (m, 2H), 7.07-6.96 (m, 2H), 6.70 (d, *J* = 7.5 Hz, 1H), 6.44 (s, 1H), 5.74 (s, 1H), 2.27 (s, 3H), 2.15 (s, 3H).

**2-((2-fluoro-3-(trifluoromethyl)phenyl)(1H-imidazol-5-yl)methyl)pyridine, 393:**

**(Method: A)**

**¹H NMR** (300 MHz, CD₃OD): δ 8.48 (d, *J* = 4.8 Hz, 1H), 7.79 (t, *J* = 7.8, Hz, 1H), 7.69 (s, 1H), 7.58 (t, *J* = 6.3 Hz, 1H), 7.42 (t, *J* = 6.3 Hz, 1H), 7.38-7.24 (m, 3H), 6.66 (s, I H), 5.92 (s, I H).

**3-((2-fluoro-3-(trifluoromethyl)phenyl)(1H-imidazol-5-yl)methyl)pyridine, 394:**

**(Method: A)**

**¹H NMR** (300 MHz, CD₃OD): δ 8.43(dd, *J* = 1.5, 5.1 Hz, 1H), 8.40 (d, *J* = 2.4 Hz, 1H), 7.71 (s, 1H), 7.69-7.65 (m, 1H), 7.62 (t, *J* = 7.8 Hz, 1H), 7.44-7.38 (m, 2H), 7.31 (t, *J* = 7.8 Hz, 1H), 6.68 (s, 1H), 5.87 (s, 1H).

**4-((2-fluoro-3-(trifluoromethyl)phenyl)(1H-imidazol-5-yl)methyl)pyridine, 395:**

**(Method: A)**

**¹H NMR** (300 MHz, CD₃OD): δ 8.48-8.46 (m, 2H), 7.71 (s, 1H), 7.63 (t, *J* = 6.6 Hz, 1H), 7.42 (t, *J* = 6.9 Hz, 1H), 7.34 (d, *J* = 7.8 Hz, 1H), 7.29-7.25 (m, 2H), 6.72 (s, 1H), 5.85 (s, 1H).

**2-((2,3-dichlorophenyl)(1H-imidazol-5-yl)methyl)pyridine, 049:**

**(Method: A)**

**¹H NMR** (300 MHz, CD₃OD): δ 8.48-8.45 (m, 1H), 7.76 (td, *J* = 7.8, 9.6 Hz, 1H), 7.68 (s, 1H), 7.44 (dd, *J* = 1.8, 8.4 Hz, 1H), 7.31-7.26 (m, 1H), 7.23 (t, *J* = 8.1 Hz, 1H), 7.16 (d, *J*= 7.8 Hz, 1H), 6.99 (dd, *J* = 6.3, 7.5 Hz, 1H), 6.53 (s, 1H), 6.04 (s, 1H).

**3-((2,3-dichlorophenyl)(1H-imidazol-5-yl)methyl)pyridine, 050:**

**(Method: A)**

**¹H NMR** (300 MHz, CD₃OD): δ 8.43 (dd, *J* = 1.8, 5.1 Hz, 1H), 8.35 (d, *J* = 7 Hz, 1H), 7.70 (s, 1H), 7.62-7.58 (m, 1H), 7.47 (dd, *J* = 1.5, 7.8 Hz, 1H), 7.39 (dd, *J* = 5.1, 8.1 Hz, 1H), 7.25 (t, *J* = 8.1 Hz, 1H), 7.05 (dd, *J* = 1.5, 7.8 Hz, 1H), 6.56 (s, 1H), 5.96 (s, 1H).

**4-((2,3-dichlorophenyl)(1H-imidazol-5-yl)methyl)pyridine, 051 :**

**(Method: A)**

**¹H NMR** (300 MHz, CD₃OD): δ 8.46-8.44 (m, 2H), 7.70 (s, 1H), 7.48 (dd, *J*= 1.8, 8.1 Hz, 1H), 7.24 (t, *J* = 8.1 Hz, 1H), 7.21-7.19 (m, 2H), 7.05 (dd, *J* = 1.5, 7.5 Hz, 1H), 6.60 (s, 1H), 5.95 (s, 1H).

**2-((3-chloro-2-methylphenyl)(1H-imidazol-5-yl)methyl)pyridine, 984:**

**(Method: A)**

**¹H NMR** (300 MHz, CD₃OD): δ 8.49-8.47 (m, 1H), 7.8 (s, 1H) 7.81-7.75 (m, 1H), 7.33-7.28 (m, 2H), 7.14-706 (m, 2H), 6.80 (d, *J*= 9 Hz, 1H), 6.51 (s, 1H), 5.85 (s, 1H), 2.31 (s, 3H).

**4-((3-chloro-2-methylphenyl)(1H-imidazol-5-yl)methyl)pyridine, 983:**

**(Method: A)**

**¹H NMR** (300 MHz, CD₃OD): δ 8.46-8.44 (m, 2H), 7.70 (s, 1H), 7.30 (d, *J* = 9 Hz, 1H), 7.19-7.17(m, 2H), 7.10 (t, *J* = 6Hz, 1H), 6.82 (d, *J* = 9Hz, 1H), 6.51 (s, 1H), 5.75 (s, 1H), 2.31 (s, 3H).

**2-((3-fluoro-2-methylphenyl)(1H-imidazol-5-yl)methyl)pyridine, 119:**

**(Method: B)**

**¹H NMR** (300 MHz, CD₃OD): δ 8.49-8.47 (m, 1H), 7.80-7.74 (m, 2H), 7.32-7.28 (m, 1H), 7.14-7.07 (m, 2H), 6.95 (t, *J* = 8.7 Hz, I H), 6.67 (d, *J* = 7.5 Hz, 1H), 6.49 (s, 1H), 5.80 (s, 1H), 2.16 (s, 3H).

**4-((3-fluoro-2-methylphenyl)(1H-imidazol-5-yl)methyl)pyridine, 330:**

**(Method: B)**

**¹H NMR** (300 MHz, CD₃OD): δ 8.45-8.43 (m, 2H), 7.69 (s, 1H), 7.20-7.09 (m, 3H), 6.97 (t, *J* = 8.4 Hz, 1H), 6.71 (d, *J* = 8.1 Hz, 1H), 6.51 (s, 1H), 5.70 (s, 1H), 2.15 (s, 3H).

**2-((2,3-difluorophenyl)(1H-imidazol-5-yl)methyl)pyridine, 419:**

**(Method: A)**

**¹H NMR** (300 MHz, CD₃OD): δ 8.47 (d, *J*= 4.2 Hz, 1H), 7.78 (td, *J*= 7.8 Hz, 9.3 Hz, 1H), 7.68 (s, 1H), 7.32-7.23 (m, 2H), 7.19-7.06 (m, 2H), 6.91-6.86 (m, 1H), 6.63 (s, 1H), 5.88 (s, 1H).

**3-((2,3-difluorophenyl)(1H-imidazol-5-yl)methyl)pyridine, 519:**

**(Method: A)**

**¹H NMR** (300 MHz, CD₃OD): δ 8.43 (d, *J* = 6 Hz, 1H), 8.39 (d, *J* = 3 Hz, 1H), 7.70 (s, 1H), 7.68 (d, *J* = 9 Hz, 1H), 7.40 (dd, *J* = 6 Hz, 9 Hz, 2H), 7.20-7.11 (m, 2H), 6.94 (t, *J* = 9 Hz, I H), 6.66 (s, I H), 5.82 (s, 1H).

**4-((2,3-difluorophenyl)(1H-imidazol-5-yl)methyl)pyridine, 665:**

**(Method: A)**

**¹H NMR** (300 MHz, CD₃OD): δ 8.47-8.45 (m, 2H), 7.70 (s, 1H), 7.27-7.25 (m, 2H), 7.21-7.11 (m, 2H), 6.92 (t, *J* = 6 Hz, 1H), 6.70 (s, 1H), 5.80 (s, 1H).

**2-((2-methylphenyl)(1H-imidazol-5-yl)methyl)pyridine, 668:**

**(Method: B)**

**¹H NMR** (300 MHz, CD₃OD): δ 8.46 (d, *J*= 4.8 Hz, 1H), 7.76 (td, *J*= 9Hz, 14.4 Hz, 1H), 7.66 (s, 1H), 7.31-7.27 (m, 1H), 7.19-7.09 (m, 3H), 6.84 (d, 6.6Hz, 1H), 6.40 (s, 1H), 5.77 (s, 1H), 2.25(s, 1H).

**3-((2-methylphenyl)(1H-imidazol-5-yl)methyl)pyridine, 669:**

**(Method: B)**

**¹H NMR** (300 MHz, CD₃OD): δ 8.41-8.39 (m, 1H), 8.31 (s, 1H), 7.68 (s, 1H), 7.57 (d, *J* = 9 Hz, 1H), 7.38 (dd, *J* = 3, 6 Hz, 1H), 7.18-7.12 (m, 3H), 6.87 (d, *J* = 6Hz, 1H), 6.43 (s, 1H), 5.71 (s, 1H), 2.26 (s, 3 H).

**4-((2-methylphenyl)(1H-imidazol-5-yl)methyl)pyridine, 742:**

**(Method: B)**

**¹H NMR** (300 MHz, CD₃OD): δ 8.44-8.42 (m, 2H), 7.68 (s, 1H), 7.19-7.17 (m, 2H), 7.16-7.11(m, 2H), 7.19 (d, *J* = 9Hz, 1H), 6.47 (s, 1H), 5.68 (s, 1H), 2.25 (s, 3H).

**2-((2-fluorophenyl)(1H-imidazol-5-yl)methyl)pyridine, 667:**

**(Method: A)**

**¹H NMR** (300 MHz, CD₃OD): δ 8.47-8.45 (d, *J*= 9 Hz, 1H), 7.76 (td, *J* = 8.1 Hz, 9.9 Hz, 1H), 7.66 (s, 1H), 7.31-7.20 (m, 3 H), 7.10-7.07 (m, 3 H), 6.58 (s, 1H), 6.86 (s, 1H).

**3-((2-fluorophenyl)(1H-imidazol-5-yl)methyl)pyridine, 982:**

**(Method: A)**

**¹H NMR** (300 MHz. CD₃OD): δ 8.41-8.39 (m, 1H), 8.67 (d, *J* = 2.1Hz, 1H), 7.69(s, 1H), 7.67 (d, *J* = 6 Hz, 1H), 7.35 ( dd, *J* = 12.6 Hz, 12.9 Hz, 2H), 7.30-7.26 (m, 2H), 7.10-7.16 (m, 3H), 6.61 (s, 1H), 5.79 (s, 1H).

**4-((2-fluorophenyl)(1H-imidazol-5-yl)methyl)pyridine, 666:**

**(Method: A)**

**¹H NMR** (300 MHz, CD₃OD): δ 8.45-8.43 (m, 2H), 7.69 (s, 1H), 7.33-7.28 (m, 2H), 7.24-7.22(m, 2H), 7.14-7.06 (m, 2H), 7.04 (s, 1H), 6.65 (s, 1H), 5.78 (s, 1H).

**2-((3-fluorophenyl)(1H-imidazol-5-yl)methyl)pyridine, 118:**

**(Method: B)**

**¹H NMR** (300 MHz, CD₃OD): δ 8.48-8.46 (m, 1H), 7.77 (ddd, *J* = 1.8, 7.8, 13.5 Hz, 1H), 7.66 (s, 1H), 7.33-7.26 (m, 3H), 7.05 (d, *J* = 7.5 Hz, 1H), 6.98-6.90 (m, 2H), 6.64 (s, 1H), 5.59 (s, 1H).

**3-((3-fluorophenyl)(1H-imidazol-5-yl)methyl)pyridine, 447:**

**(Method: B)**

**¹H NMR** (300 MHz, CD₃OD): δ 8.41-8.39 (m, 2H), 7.68 (s, I H), 7.67-7.63 (m, 1H), 7.39-7.28 (m, 2H), 7.04-6.91 (m, 3H), 6.64 (s, 1H), 5.56 (s. 1H).

**4-((3-fluorophenyl)(1H-imidazol-5-yl)methyl)pyridine, 298:**

**(Method: B)**

**¹H NMR** (300 MHz, CD₃OD): δ 8.446-8-44 (m, 2H), 7.69 (s, I H), 7.35-7.31 (m, 1H), 7.27-7.25 (m, 2H), 7.03-6.90 (m, 3H), 6.68 (s, 1H), 5.53 (s, 1H).

**2-((pheyl)(1H-imidazol-5-yl)methyl)pyridine, 302:**

**(Method: B)**

**¹H NMR (300 MHz, CD₃OD)**: δ 8.47 (d, *J* = 4.8 Hz, 1H), 7.79-7.73 (m, 2H), 7.32-7.19 (m, 7H), 6.62 (s, 1H), 5.59 (s, 1H).

**3-((phenyl)(1H-imidazol-5-yl)methyl)pyridine, 891:**

**(Method: B)**

**¹H NMR** (300 MHz, CD₃OD): δ 8.39-8.36 (m, 2H), 7.67 (s, 1H), 7.66-7.62 (m, 1H), 7.39-7.18 (m, 6H), 6.57 (s, 1H), 5.52 (s, 1H).

**4-((phenyl)(1H-imidazol-5-yl)methyl)pyridine, 892:**

**(Method: B)**

**¹H NMR** (300 MHz, CD₃OD): δ 8.42-8.40 (m, 2H), 7.66 (s, I H), 7.33-7.18 (m, 7H), 6.60 (s, 1H), 5.48 (s, 1H).

**4-((3-chloro-2-fluorophenyl)(thiophen-2-yl)methyl)-1H-imidazole, 056:**

**(Method: A)**

**¹H NMR** (300 MHz, CDCl₃): δ 7.63 (s, I H), 7.35 (dd, *J* = 2.1 Hz, 7.5 Hz, 9.9 Hz, 1H), 7.28-7.26 (m, 1H), 7.14-7.04 (m, 2H), 6.94 (dd, *J*= 3.3 Hz, 5.1 Hz, I H), 6.78 (d, *J* = 3.6 Hz, 1H), 6.71 (s, 1H), 5.96 (s, I H).

**3-((2-chloro-3-fluorophenyl)(1H-imidazol-4-yl)methyl)pyridine, 322:**

**(Method: A)**

**¹H NMR** (300 MHz, CDCl₃): δ 8.47-8.45 (m, 2H), 7.56 (s, 1H), 7.50 (dt, *J*= 7.8, 2.1 Hz, 1H), 7.25-7.13 (m, 2H), 7.04 (td, *J*= 1.8 Hz, 8.7 Hz, 1H), 6.94 (d, *J*= 7.8 Hz, 1H), 6.48 (s, I H), 5.88 (s, I H).

**4-((2-chloro-3-fluorophenyl)(4,5-diisopropylthiophen-2-yl)methyl)-1H-imidazole, 332:**

**(Method: A)**

**¹H NMR** (300 MHz, CDCl₃): δ 7.44 (s, 1H), 7.19-7.09 (m, 2H), 7.03-6.97 (m, 1H), 6.66 (s, 1H), 6.57 (s, I H), 6.02 (s, 1H), 3.22 (heptet, *J*= 6.9 Hz, I H), 2.94 (heptet, *J*= 6.9 Hz, 1H), 1.22 (d, *J*= 6.9 Hz, 6H), 1.13-1.10 (m, 6H).

**4-((2-chloro-3-fluorophenyl)(4-isopropylthiophen-2-yl)methyl)-1H-imidazole, 335:**

**(Method: A)**

**¹H NMR** (300 MHz, CDCl₃): δ 7.55 (s, 1H), 7.21-7.00 (m, 3 H), 6.79-6.78 (m, 1H), 6.71-6.70 (m, 2H), 6.08 (s, 1H), 2.85 (heptet, *J* = 6.6 Hz, 1H), 1.19 (d, *J* = 6.6 Hz, 6H).

**4-((2-chloro-3-fluorophenyl)(5-isopropylthiophen-2-yl)methyl)-1H-imidazole, 337:**

**(Method: A)**

**¹H NMR** (300 MHz, CDCl₃): δ 7.52 (s, 1H), 7.2-6.99 (m, 3H), 6.69 (s, 1H), 6.6-6.57 (m, 2H), 6.04 (s, 1H), 3.07 (heptet, *J* = 6.6 Hz, I H), 1.27 (d, *J*= 6.6 Hz, 6H).

**Biological Data**

**Receptor Selection and Amplification Technology (RSAT) assay**

The RSAT assay measures a receptor-mediated loss of contact inhibition that results in selective proliferation of receptor-containing cells in a mixed population of confluent cells. The increase in cell number is assessed with an appropriate transfected marker gene such as β-galactosidase, the activity of which can be easily measured in a 96-well format. Receptors that activate the G protein, Gq, elicit this response. Alpha2 receptors, which normally couple to Gi, activate the RSAT response when coexpressed with a hybrid Gq protein that has a Gi receptor recognition domain, called Gq/i5.

NIH-3T3 cells are plated at a density of 2x106 cells in 15 cm dishes and maintained in Dulbecco's modified Eagle's medium supplemented with 10% calf serum. One day later, cells are cotransfected by calcium phosphate precipitation with mammalian expression plasmids encoding p-SV-β-galactosidase (5-10 µg), receptor (1-2 µg) and G protein (1-2 µg). 40 µg salmon sperm DNA may also be included in the transfection mixture. Fresh media is added on the following day and 1-2 days later cells are harvested and frozen in 50 assay aliquots. Cells are thawed and 100 µl added to 100 µl aliquots of various concentrations of drugs in triplicate in 96-well dishes. Incubations continue 72-96 hr at 37 °C. After washing with phosphate-buffered saline, β-galactosidase enzyme activity is determined by adding 200 µl of the chromogenic substrate (consisting of 3.5 mM o-nitrophenyl-β-D-galactopyranoside and 0.5% nonidet P-40 in phosphate buffered saline), incubating overnight at 30 °C and measuring optical density at 420 nm. The absorbance is a measure of enzyme activity, which depends on cell number and reflects a receptor-mediated cell proliferation. The efficacy or intrinsic activity is calculated as a ratio of the maximal effect of the drug to the maximal effect of a standard full agonist for each receptor subtype. Brimonidine, also called UK 14304, the chemical structure of which is shown below, is used as the standard agonist for the alpha_{2A}, alpha_{2B} and alpha_{2C} receptors. The EC₅₀ is the concentration at which the drug effect is half of its maximal effect.

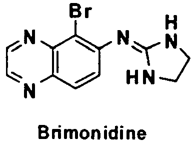

The results of the RSAT assay with several exemplary compounds of the invention are disclosed in **Table I** above together with the chemical formulas of these exemplary compounds. EC₅₀ values are nanomolar. ND stands for "not determinable" at concentrations less than 10 micromolar. 1A stands for "intrinsic activity."

**Table 1**

| **Biological Data: Intrinsic Activity** potency nM; efficacy (EC50) | | | |
|---|---|---|---|
| Structure | Alpha 2A | Alpha 2B | Alpha 2C |
| 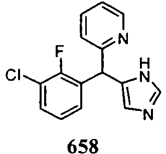 | ND (0.07) | 32 (1.21) | 177 (0.49) |
| 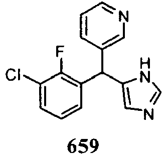 | ND (0.28) | 5.2 (1.34) | 64 (0.55) |
| 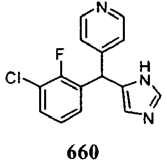 | ND (0.09) | 40 (1.02) | 340 (0.60) |
| 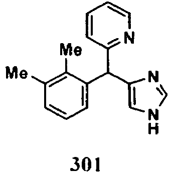 | 1932 (0.39) | 0.92 (1.22) | 356 (0.64) |
| 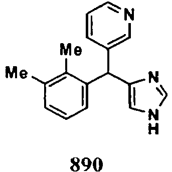 | ND (0.19) | 0.33 (1.25) | 14 (0.55) |
| 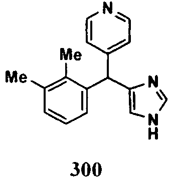 | 1511 (0.32) | 4.4 (1.07) | 605 (0.49) |
| 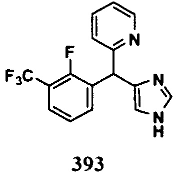 | ND (0.08) | 39 (1.1) | 507 (1.09) |
| 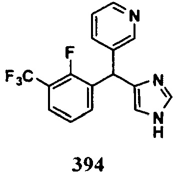 | ND (0.23) | 5.2 (1.24) | 32 (0.42) |
| 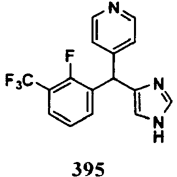 | ND (0.14) | 45 (1.29) | ND (0.29) |
| 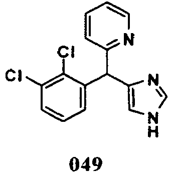 | ND (0.21) | 8.7 (.85) | 784 (0.39) |
| 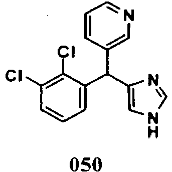 | 605 (0.37) | 1.62 (1.15) | 112 (0.87) |
| 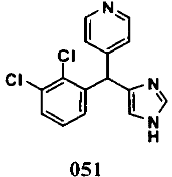 | ND (0.18) | 3.2 (1.31) | 97 (0.72) |
| 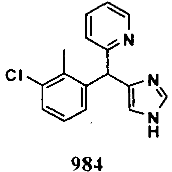 | ND (0.12) | 7.8 (1.39) | 495 (0.53) |
| 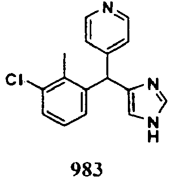 | ND (0.11) | 10.3 (1.10) | 83 (0.46) |
| 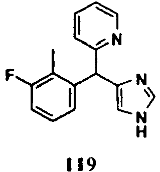 | ND (0.04) | 36 (1.07) | 440 (0.38) |
| 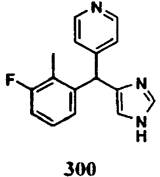 | ND (0.03) | 72 (1.17) | ND (0.27) |
| 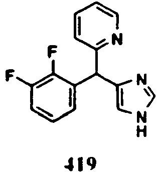 | ND (0.05) | 405 (1.08) | ND (0.16) |
| 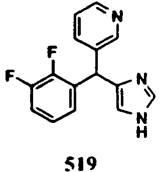 | ND (0.05) | 68 (0.93) | 656 (0.85) |
| 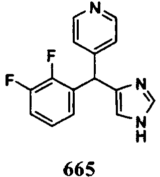 | ND (0.01) | 837 (0.70) | 2486 (1.02) |
| 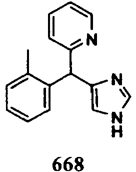 | ND (0.03) | 177 (0.92) | 213 (0.57) |
| 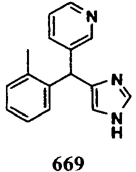 | ND (0.06) | 259 (0.61) | ND (0.29) |
| 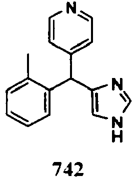 | ND (0.06) | 63 (0.82) | 265 (1.08) |
| 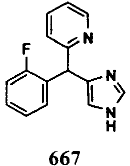 | ND (0.04) | 993 (0.56) | 2081 (0.43) |
| 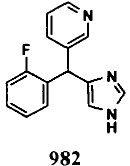 | ND (0.7) | 251 (0.60) | 2475 (0.55) |
| 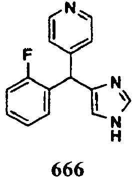 | ND (0.04) | 350 (0.78) | 365 (0.48) |
| 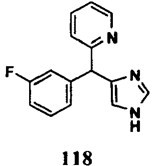 | ND (0.14) | 273 (0.64) | ND (0.25) |
| 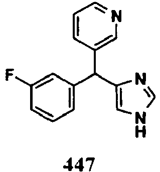 | ND (0.05) | 222 (1.01) | 3643 (0.47) |
| 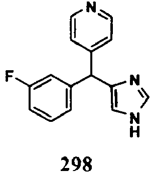 | ND (0.04) | 562 (0.93) | ND (0.23) |
| 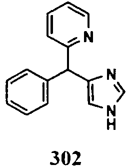 | ND (0.04) | 469 (0.87) | ND (0.09) |
| 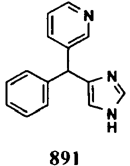 | ND (0.11) | 190 (0.72) | ND (0.16) |
| 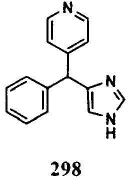 | ND (0.10) | 309 (0.69) | ND (0.14) |
| 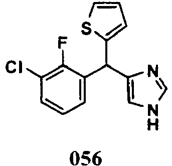 | nd (0.21) | 46 (0.93) | nd (0.28) |
| 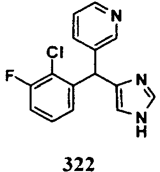 | 3373 (0.32) | 4.8 (0.98) | 88 (0.87) |
| 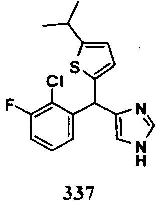 | nd (0.06) | 75 (0.40) | nd (0.23) |

The foregoing description details specific methods and compositions that can be employed to practice the present invention, and represents the best mode contemplated. However, it is apparent for one of ordinary skill in the art that further compounds with the desired pharmacological properties can be prepared in an analogous manner, and that the disclosed compounds can also be obtained from different starting compounds via different chemical reactions. Similarly, different pharmaceutical compositions may be prepared and used with substantially the same result. Thus, however detailed the foregoing may appear in text, it should not be construed as limiting the overall scope hereof; rather, the ambit of the present invention is to be governed only by the lawful construction of the claims.

## Claims

1. A compound of the formula 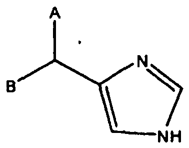 wherein
A is pyridyl, thienyl, furyl, pyrrolyl, pyrrolidinyl, imidazolyl, oxazolyl, pyrazolyl, pyrimidinyl, or pyrazinyl having 0, 1, 2, or 3 substituents;
B is phenyl having 0, 1, 2, or 3 substituents; and
each substituent is independently alkyl having from 1 to 8 carbon atoms, -F, -Cl, -Br, -CH₂OH, aminomethyl, -CH₂CN, -CF₃, or acyl having from 1 to 8 carbons.

2. The compound of claim 1 wherein A is unsubstituted or has an isopropyl substituent.

3. The compound of claim 1 wherein each substituent of B is -F, -Cl, -CH₃, or -CF₃.

4. The compound of claim 1 wherein A is pyridinyl.

5. The compound of claim 1 wherein A is thienyl.

6. The compound of claim 1 selected from

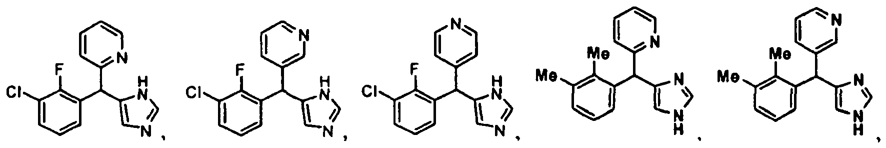

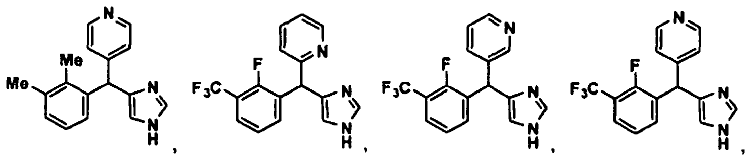

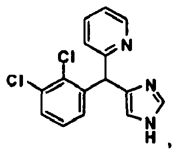

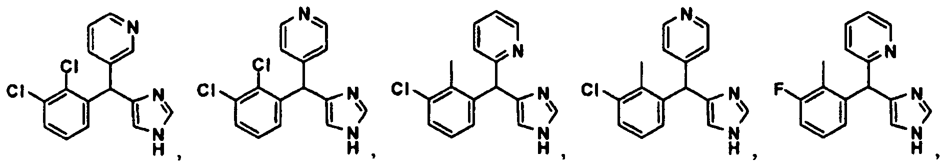

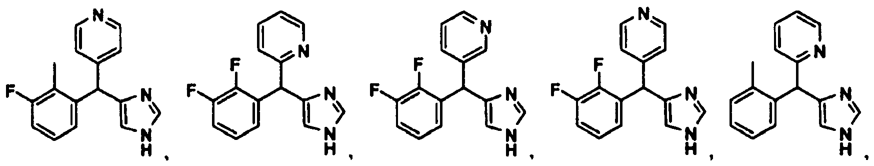

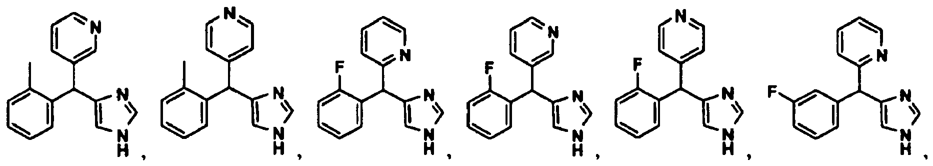

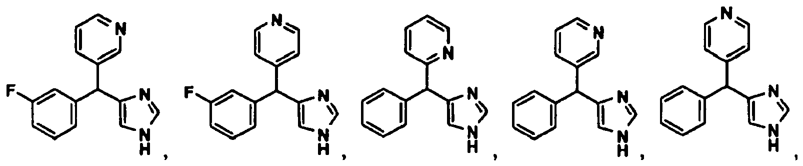

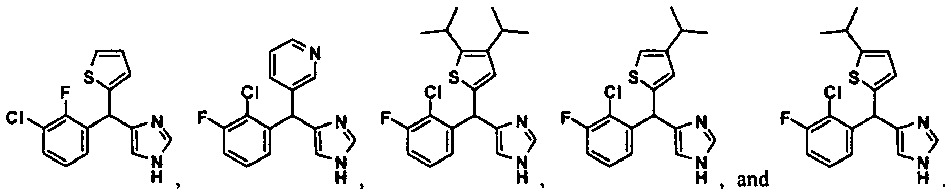

7. A compound according to any preceding claim for use in the treatment of pain in a mammal.

8. Use of a compound according to any one of claims 1 to 6 in the manufacture of a medicament for the treatment of pain.

9. A pharmaceutically acceptable composition comprising a compound according to any one of claims 1 to 6.

10. A kit comprising a package containing the composition of claim 9, and a label indicating the use of the composition for the treatment of pain.

## Patentansprüche

1. Verbindung der Formel: 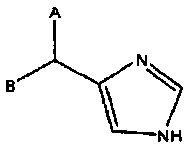 worin
A Pyridyl, Thienyl, Furyl, Pyrrolyl, Pyrrolidinyl, Imidazolyl, Oxazolyl, Pyrazolyl, Pyrimidinyl oder Pyrazinyl mit 0, 1, 2 oder 3 Substituenten ist;
B Phenyl mit 0, 1, 2 oder 3 Substituenten ist; und
jeder Substituent unabhängig Alkyl mit 1 bis 8 Kohlenstoffatomen, -F, -Cl, -Br, -CH₂OH, Aminomethyl, -CH₂CN, -CF₃ oder Acyl mit 1 bis 8 Kohlenstoffen ist.

2. Verbindung gemäss Anspruch 1, worin A unsubstituiert ist oder einen Isopropylsubstituenten aufweist.

3. Verbindung gemäss Anspruch 1, worin jeder Substituent von B -F, -Cl, -CH₃ oder -CF₃ ist.

4. Verbindung gemäss Anspruch 1, worin A Pyridinyl ist.

5. Verbindung gemäss Anspruch 1, worin A Thienyl ist.

6. Verbindung gemäss Anspruch 1, ausgewählt aus: 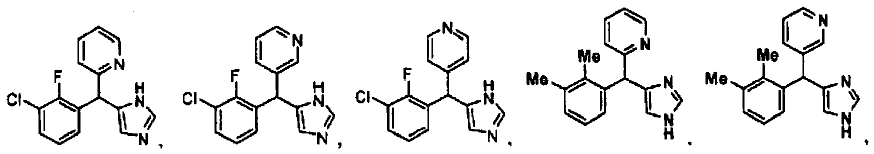 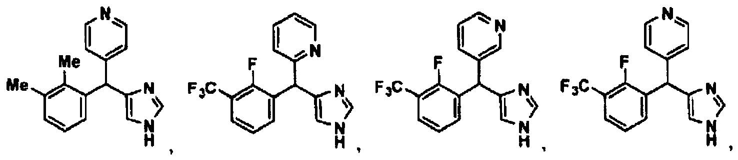 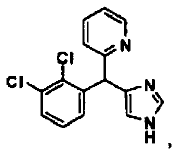 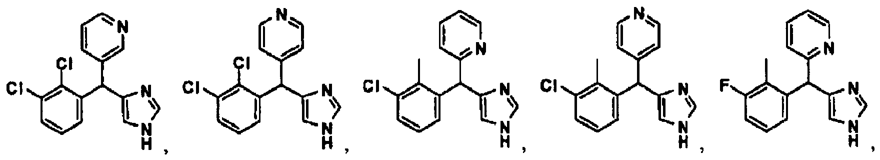 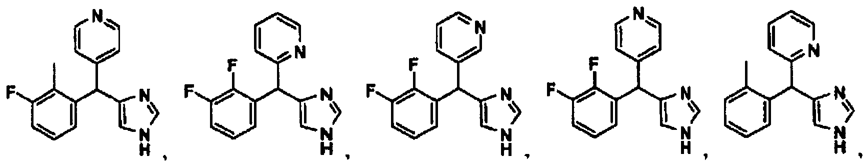 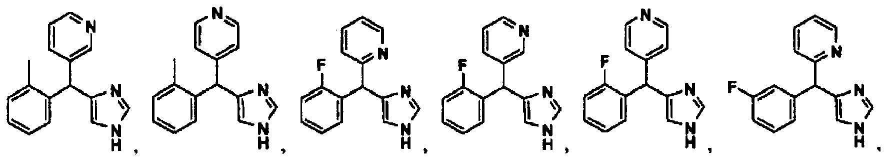 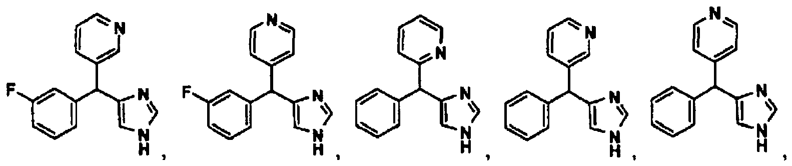 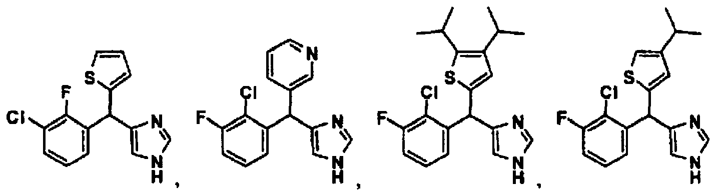 und

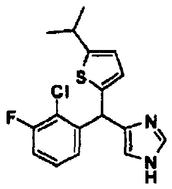

7. Verbindung gemäss irgendeinem der vorhergehenden Ansprüche zur Verwendung bei der Behandlung von Schmerz bei einem Säuger.

8. Verwendung einer Verbindung gemäss irgendeinem der Ansprüche 1 bis 6 bei der Herstellung eines Medikaments zur Behandlung von Schmerz.

9. Pharmazeutisch annehmbare Zusammensetzung, umfassend eine Verbindung gemäss irgendeinem der Ansprüche 1 bis 6.

10. Kit, umfassend eine Packung, die die Zusammensetzung gemäss Anspruch 9 und ein Etikett, das die Verwendung der Zusammensetzung bei der Behandlung von Schmerz anzeigt, enthält.

## Revendications

1. Composé de la formule 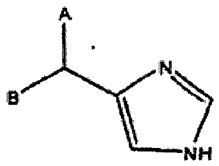 dans laquelle
A est un pyridyle, un thiényle, un furyle, un pyrrolyle, un pyrrolidinyle, un imidazolyle, un oxazolyle, un pyrazolyle, un pyrimidinyle, ou un pyrazinyle ayant 0, 1, 2, ou 3 substituant(s) ;
B est un phényle ayant 0, 1, 2, ou 3 substituant(s) ; et
chaque substituant est indépendamment un alkyle ayant de 1 à 8 atomes de carbone, -F, -Cl, -Br, -CH₂OH, un aminométhyle, -CH₂CN, -CF₃, ou un acyle ayant de 1 à 8 atomes de carbone.

2. Composé selon la revendication 1 dans lequel A est non substitué ou a un substituant isopropyle.

3. Composé selon la revendication 1 dans lequel chaque substituant de B est -F, -Cl, -CH₃ ou -CF₃.

4. Composé selon la revendication 1 dans lequel A est un pyridinyle.

5. Composé selon la revendication 1 dans lequel A est un thiényle.

6. Composé selon la revendication 1 choisi parmi

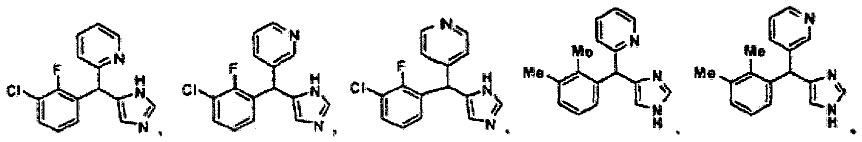

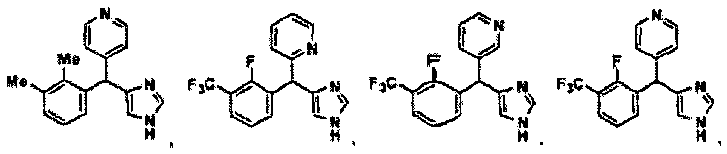

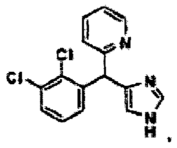

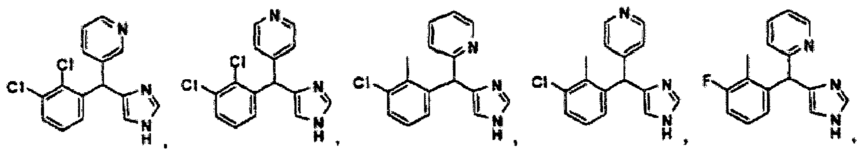

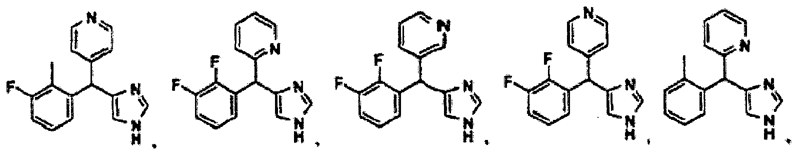

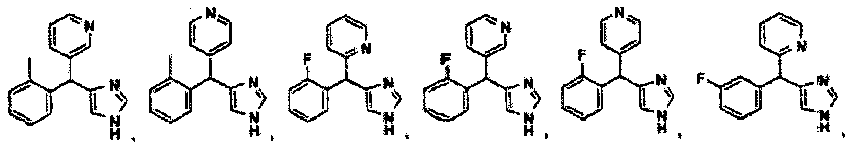

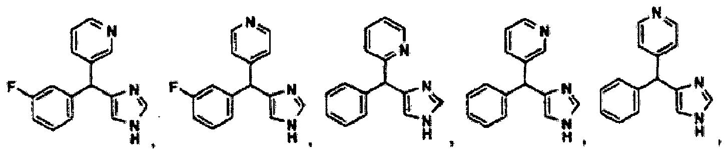

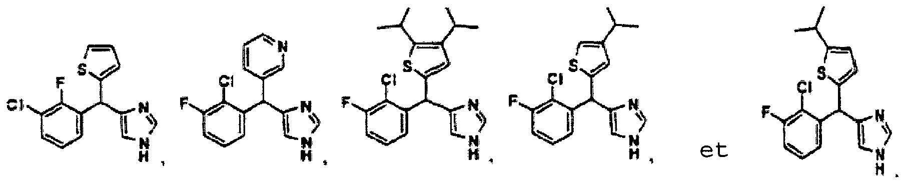

7. Composé selon une quelconque revendication précédente pour une utilisation dans le traitement d'une douleur chez un mammifère.

8. Utilisation d'un composé selon l'une quelconque des revendications 1 à 6 dans la fabrication d'un médicament pour le traitement d'une douleur.

9. Composition pharmaceutiquement acceptable comprenant un composé selon l'une quelconque des revendications 1 à 6.

10. Trousse comprenant un conditionnement contenant la composition selon la revendication 9, et une étiquette indiquant l'utilisation de la composition pour le traitement d'une douleur.
